# EUROPEAN PATENT APPLICATION

(11) **EP 2 407 199 A1**
(43) Date of publication of application: **18.01.2012**
(21) Application number: 10750520.8
(22) Date of filing: 05.03.2010
(51) Int. Cl.: A61M 25/00

(54) **CATHETER AND METHOD OF MANUFACTURING CATHETER**

(30) Priority: 09.03.2009 JP 2009054806
(71) Applicant: SUMITOMO BAKELITE CO., LTD., Tokyo 140-0002 (JP)
(72) Inventor: TANAKA, Hayao, Akita-shi Akita 011-8510 (JP); HAYASHI, Hiroyuki, Tokyo 140-0002 (JP)
(74) Representative: Vossius, Corinna
(86) International application number: PCT/JP2010/001532
(87) International publication number: WO 2010/103762

(57) **Abstract**

A main lumen (20) and sub-lumens (30) having a diameter smaller than that of the main lumen (20) are respectively formed in a sheath (16) of a catheter (10) in the longitudinal direction thereof so as to extend therethrough. An operating wire (40) is laid through each sub-lumen (30) in a slidable manner, with the end thereof fixed to the distal end section (15) of the sheath (16). The sheath (16) is configured a first resin layer (61) and a second resin layer (62) having a hardness higher than that of the first resin layer (61), stacked in the thickness-wise direction, each of which being composed of a resin material. In the sheath(16), the thickness of the second resin layer (62) increases from the distal end side towards the proximal end side.

## Description

### TECHNICAL FIELD

The present invention relates to a catheter, and a method of manufacturing a catheter.

### BACKGROUND ART

Recently, there has been proposed a catheter, the direction of insertion of which into a body cavity is controllable by allowing the distal end section thereof to bend. As one mode of bending of the distal end of the catheter, a known catheter is configured to operate a wire fixed to the distal end, at the proximal end side (see Patent Document 1 below).
The wire (operating wire) laid through a lumen of the body of the catheter (tubular body) has a predetermined rigidity, and the distal end of the tubular body is bent by pushing the wire. The tubular body herein is sectioned in the longitudinal direction into about four or five sections aligned in series, having stepwisely different flexibility, wherein the rigidity increases towards the proximal end side, and conversely the flexibility increases towards the distal end side. According to the description, the catheter is able to largely bend the distal end section thereof when the wire is pushed, while keeping a predetermined level of resilience.

### RELATED DOCUMENTS

### PATENT DOCUMENT

[Patent Document 1] Published Japanese Translation of PCT International Publication for Patent Application No. 2007-507305

### DISCLOSURE OF THE INVENTION

In recent years, diameter of catheters has progressively been shrunk, typically in view of insertability into blood vessel, and those having an outer diameter of 1 mm or smaller have been supplied. In this case, from the viewpoint of ensuring a predetermined inner diameter for a main lumen through which any drug and so forth is delivered or any optical component is inserted, it is desired for a sub-lumen, through which an operating wire is laid, to have an inner diameter of as very small as 10 to several tens micrometers.
In such recent catheters, represented by the catheter disclosed in Patent Document 1, having the flexibility stepwisely varied over a plurality of sections aligned in the longitudinal direction thereof, it may be difficult to ensure subtle manipulation through the operating wire. This is because bendability of the catheter is discontinuous at the boundary portions where the flexibility stepwisely varies, although bending rigidity (geometrical moment of inertia) of the tubular body is generally known to be proportional to the fourth power of diameter. For this reason, relation of the length of pushing or pulling of the operating wire, with the amount of flexion of the distal end may be complicated, and it may therefore become difficult to bend the distal end by a predetermined amount of flexion. On the other hand, any trial made on the conventional catheter so as to gradually vary the flexibility of the tubular body over a number of sections aligned in the longitudinal direction thereof, intended for varying the bendability of the tubular body in a substantially continuous manner, may suffer from problems in costs of molding and materials, because a vast variety of materials will necessarily be used.

The present invention is conceived after considering the above-described problems, and is aimed at providing a catheter which allows subtle bending manipulation through the operating wire irrespective of shrinkage of the diameter of sub-lumen, and is readily manufacturable, and a method of manufacturing the same.

A catheter of the present invention has a tubular body which includes a main lumen, and a sub-lumen having a diameter smaller than that of the main lumen, both of which being formed in a longitudinal direction so as to extend therethrough; and an operating wire laid through the sub-lumen in a slidable manner, with an end thereof fixed to a distal end section of the tubular body. The tubular body is configured by a first resin layer, and a second resin layer having a hardness higher than that of the first resin layer, stacked in the thickness-wise direction, each of which composed of a resin material, with a thickness of the second resin layer increased from a distal end side towards a proximal end side.

In a more specific embodiment of the catheter of the present invention, the thickness of the first resin layer may monotonously decrease from the distal end side towards the proximal end side of the tubular body.

In a more specific embodiment of the catheter of the present invention, a third resin layer, having an intermediate hardness between those of the first resin layer and the second resin layer, may be stacked between the first resin layer and the second resin layer.

In a more specific embodiment of the catheter of the present invention, the second resin layer may be provided in the outer circumferential side of the first resin layer.

In a more specific embodiment of the catheter of the present invention, the resin material composing the second resin layer may have a tackiness smaller than that of the resin material composing the first resin layer.

In a more specific embodiment of the catheter of the present invention, a tubular inner layer having the main lumen formed therein, and a braid layer configured by wires woven around the inner layer, may further be provided, and the first resin layer may be formed in close contact with the braid layer.

In a more specific embodiment of the catheter of the present invention, the sub-lumen having the operating wire laid therethrough may be formed so as to extend on the outer circumferential side of the first resin layer.

In a more specific embodiment of the catheter of the present invention, the first resin layer may be provided on the outer circumferential side of the second resin layer, over a partial length of the distal end side of the tubular body.

A method of manufacturing a catheter of the present invention, which has a tubular body having a main lumen, and a sub-lumen having a diameter smaller than that of the main lumen, both of which being formed in a longitudinal direction so as to extend therethrough; and an operating wire laid through the sub-lumen in a slidable manner, includes a body forming step for forming the tubular body, by co-extruding a molten first resin material, together with a molten second resin material having a hardness higher than that of the first resin material at normal temperature, and having the operating wire introduced therein, from a die, while increasing the amount of extrusion of the second resin material relative to the amount of extrusion of the first resin material.

In a more specific embodiment of the method of manufacturing a catheter of the present invention, configured to execute the body forming step, after a preliminary extrusion step in which a molten third resin material having the operating wire introduced therein was co-extruded from the die together with the molten second resin material, wherein the tubular body may be formed in the body forming step by co-extruding the first, second and third resin materials from the die, while increasing the amounts of extrusion of the second resin materials relative to the amount of extrusion of the first and third resin material.

Note that each of various constituents of the present invention may not always necessarily be configured as an independent entity, and instead a plurality of constituents may configure a single component, a single constituents may be configured by a plurality of components, a certain constituent may be a part of other constituent, and a part of certain constituent may be shared with a part of other constituent.

According to the catheter of the present invention, since the second resin material having the largest hardness is configured to increase the thickness from the distal end side towards the proximal end side thereof, so that subtle manipulation through the operating wire may be ensured, while stably ensuring both of a large resilience of the catheter and a good bendability of the distal end section at the same time.

### BRIEF DESCRIPTION OF THE DRAWINGS

The above and other objects, features and advantages of the present invention will be more apparent from the following description of certain preferred embodiments taken in conjunction with the accompanying drawings below.

FIG. 1 is a partial schematic vertical sectional view illustrating a catheter in one embodiment of the present invention;
FIG. 2 is a partial schematic vertical sectional view illustrating a catheter of a first modified example of this embodiment;
FIG. 3 is a sectional view taken along line III-III in FIG. 2;
FIG. 4 is a side elevation for explaining operations of the catheter, wherein (a) is a schematic vertical sectional view illustrating the catheter in the natural state, and (b) is a schematic vertical sectional view illustrating the catheter with the operating wire pulled;
FIG. 5 is a drawing illustrating an overall configuration of an apparatus for manufacturing a sheath;
FIG. 6 is a schematic vertical sectional view illustrating an extruder; and
FIG. 7 is a partial schematic vertical sectional view illustrating a catheter of a second modified example of this embodiment.

### DESCRIPTION OF THE EMBODIMENTS

Hereinafter, embodiments of the present invention will be explained, referring to the attached drawings. Note that all similar constituents in all drawings will be given similar reference numerals or symbols, so as to occasionally avoid repetitive explanation.
First, a catheter obtainable by a method of manufacturing of this embodiment (hereinafter, occasionally referred to as "the present method") will be outlined, followed by detailed description of the present method.

### <Catheter>

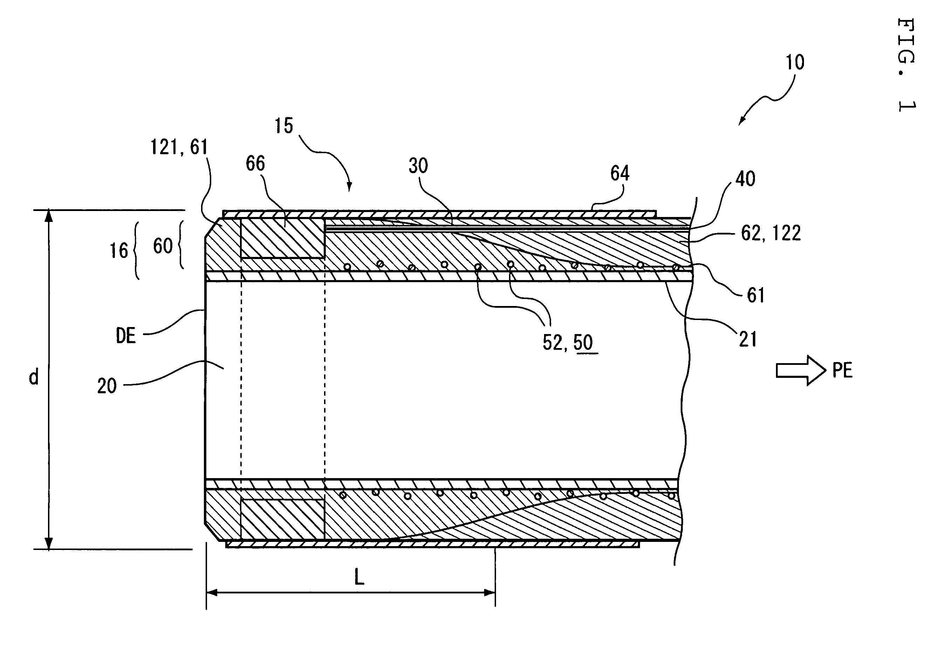

FIG. 1 is a partial schematic vertical sectional view illustrating a catheter 10 of this embodiment. The drawing illustrates a cross section of the end portion of the catheter 10 taken in the longitudinal direction. The left of the drawing corresponds to the distal end (tip) of the catheter 10, and the right corresponds to the proximal end (base). Note that the proximal end side of the catheter 10 is not illustrated in this drawing.

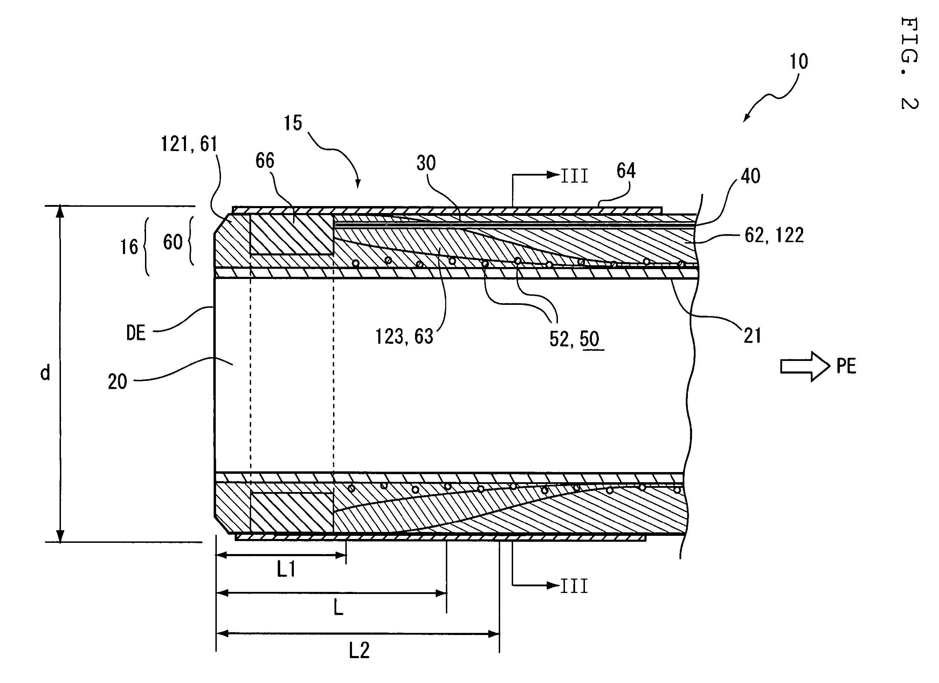

FIG. 2 is a partial schematic vertical sectional view illustrating a catheter 10 of a first modified example of this embodiment.

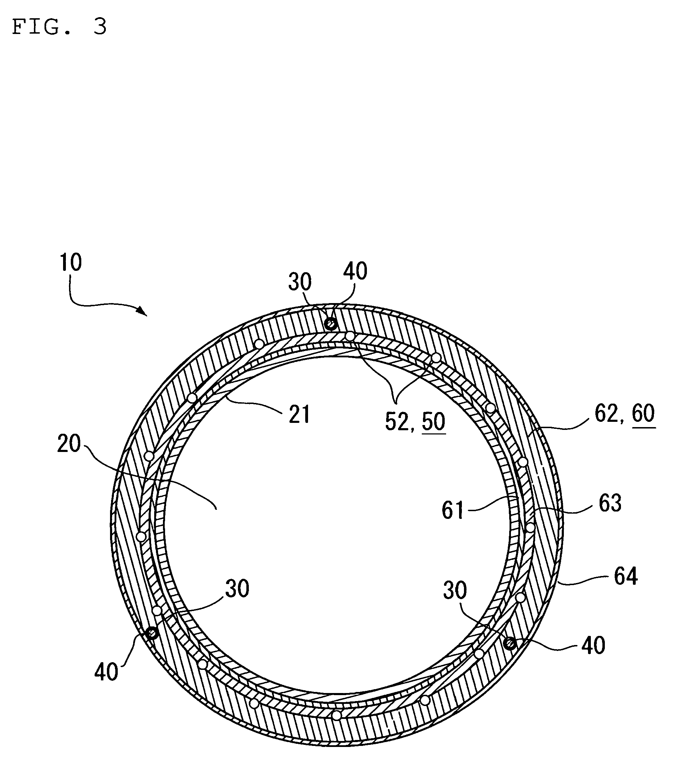

FIG. 3 is a sectional view (transverse sectional view) taken along line III-III in FIG. 2.

The catheter 10 of this embodiment has a tubular body (sheath 16) and operating wires 40.
The sheath 16 has a main lumen 20, and sub-lumens 30 having a diameter smaller than that of the main lumen 20, respectively formed in the longitudinal direction so as to extend therethrough. Each operating wire 40 is laid through each sub-lumen 30 in a slidable manner, with the end thereof fixed to the distal end section 15 of the sheath 16.
As illustrated in FIG. 1, the sheath 16 of this embodiment is configured by a first resin layer 61 and a second resin layer 62 having a hardness higher than that of the first resin layer 61, stacked in the thickness-wise direction, each of which composed of a resin material. In the sheath 16, the thickness of the second resin layer 62 increases from the distal end side towards the proximal end side.

The sheath 16 herein may have another resin layer, in addition to the first resin layer 61 and the second resin layer 62, in the thickness-wise direction or in the axial direction, as illustrated in FIG. 2. The catheter 10 of the first modified example illustrated in FIG. 2 has a third resin layer 63, in addition to the first resin layer 61 and the second resin layer 62, so as to be stacked in the thickness-wise direction.

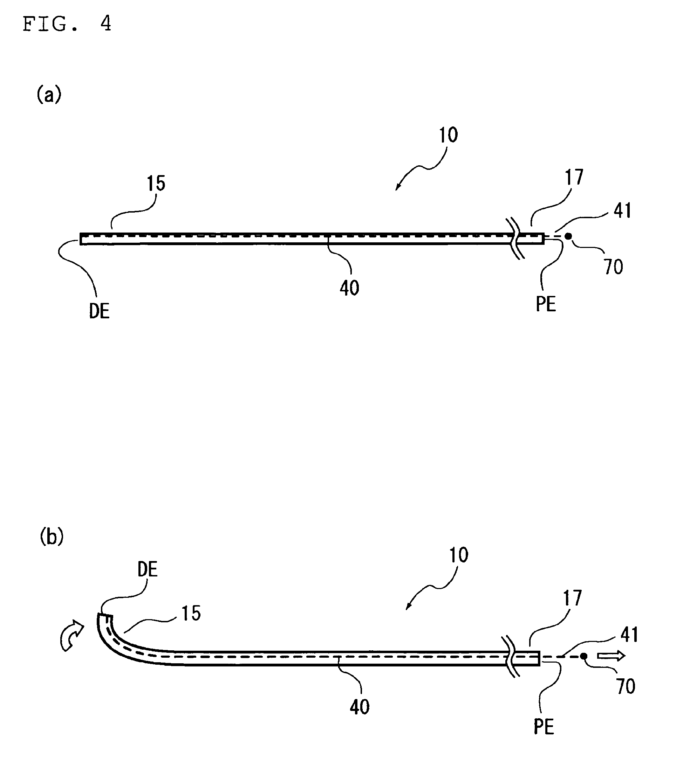

As illustrated in FIG. 3, the catheter 10 of this embodiment has a plurality of sub-lumens 30 arranged along a circumference of the main lumen 20 in a discrete manner. More specifically, three sub-lumens 30 are arranged around the main lumen 20 at 120° intervals, and each of the sub-lumens 30 has the operating wire 40 laid therethrough one-by-one in a freely slidable manner.
Note that the distal end section 15 of the catheter 10 herein means a predetermined length of region of the catheter 10 which includes the distal end DE. Similarly, the proximal end section 17 (see FIG. 4) of the catheter 10 herein means a predetermined length of region of the catheter 10 which includes the proximal end PE.
The catheter 10 of this embodiment is intended for use while being laid through an endoscope inserted into body cavity. The distal end section 15 of the catheter 10 is projected out from the end of the endoscope, so as to allow the catheter 10 to advance in a desired direction while bending the distal end section 15 thereof in the body cavity.

The sheath 16 is configured by a stack of a tubular inner layer 21 which is composed of a resin material and has a main lumen 20 formed therein, an outer layer 60 formed around the inner layer 21, and a hydrophilic coated layer 64 formed as an outermost layer of the catheter 10.

The coated layer 64 is formed so as to cover the distal end section 15 of the catheter 10, over a longitudinal portion thereof which projects out from the end of the endoscope.
The coated layer 64 is formed using a hydrophilic resin material such as polyvinyl alcohol (PVA) and polyvinylpyrrolidone, or a resin material having an lubricated outer surface, and is configured to be hydrophilic at least on the outer surface thereof.

In the vicinity of the distal end DE of the catheter 10, a ring-formed marker 66, composed of a material impermeable against radioactive ray such as X-ray, is provided. More specifically, the marker component 66 may be composed of a metal material such as platinum. The marker component 66 in this embodiment is provided around the main lumen 20 and inside the outer layer 60.

The outer layer 60 of this embodiment is configured by stacking a plurality of resin layers in the thickness-wise direction, or radial direction. More specifically, the catheter 10 of this embodiment illustrated in FIG. 1, the first and second resin layers 61, 62 are stacked in this order when viewed from the inner side of the radial direction. In other words, in the outer layer 60, the first resin layer 61 configures the inner layer, and the second resin layer 62 configures the outer layer.
On the other hand, in the catheter 10 of the modified example illustrated in FIGs. 2 and 3, the first, third, and second resin layers 61, 63, 62 are stacked in this order when viewed from the inner side of the radial direction. In other words, in the outer layer 60, the first resin layer 61 configures the innermost layer, and the second resin layer 62 configures the outermost layer provided on the outer circumferential side of the first resin layer 61.

The first to third resin layers 61 to 63 are configured by first to third resin materials 121 to 123, respectively. Each of the first to third resin materials 121 to 123 is composed of a single species, or a plurality of species of resin materials. The hardness at normal temperature decreases in the order of the second resin material 122, the third resin material 123, and down to the first resin material 121.

More specifically, in the sheath 16 of this modified example, the third resin layer 63, having an intermediate hardness between those of the first resin layer 61 and the second resin layer 62, is stacked between the first resin layer 61 and the second resin layer 62.
Note that the term "hardness", used without special comment in this embodiment, means Shore hardness.

Thermoplastic polymer may widely be adoptable to each of the first to third resin materials 121 to 123. For example, a single species, or two or more species, selected from polyimide (PI), polyamide-imide (PAI), polyethylene terephthalate (PET), polyethylene (PE), polyamide (PA), nylon elastomer, polyurethane (PU), ethylene-vinyl acetate resin (EVA), polyvinyl chloride (PVC) and polypropylene (PP), may be adoptable.

The same or different materials may be selected from those listed in the above for the first to third resin materials 121 to 123, so that they will have the hardness at normal temperature in a decreasing order of second, third and first resin materials.
For the case where the same species of polymer was selected, the hardness of the first to third resin material 121 to 123 may be varied from each other, by modifying the degree of polymerization, degree of cross-linkage or species of cross-linking agent.
The first to third resin materials 121 to 123 may be prepared by mixing a plurality of species of polymers, or may be added with an inorganic filler. The first to third resin materials 121 to 123 may have different values of hardness by varying ratio of mixing of a plurality of species of polymers, or varying species or amount of filler to be added.

It is preferable for the second resin material 122 to adopt a resin material having the lowest tackiness as compared with those of the first and the third resin materials 121, 123. It is further preferable to select the materials so that the bending rigidity at normal temperatures decreases in the order of the second, third, and first resin material.

As illustrated in FIGs. 1, 2, the thickness of the second resin layer 62 increases from the distal end side of the catheter 10 (left in FIGs. 1, 2) towards the proximal end side (right in the same). It is now defined that "the thickness of the resin layer increases from the distal end side of the catheter 10 towards the proximal end side" means that the thickness of the resin layer is kept uniform, or increases towards the proximal end side herein. Note that the description does not exclude the case where there is some decrease in the thickness of the resin layer towards the proximal end side only in a local longitudinal range, so long as functions of the present invention will not be impaired.

In the catheter 10 of this embodiment, the thickness of the first resin layer 61 monotonously decreases from the distal end side towards the proximal end side of the sheath 16.
Note that "the thickness of the resin layer monotonously decreases (increases)" herein means that the thickness of the resin layer is kept constant, or decreases (increases).

The distal end section 15 of the catheter 10 of this embodiment illustrated in FIG. 1 has a ratio of the thickness of the first resin layer 61 relative to the thickness of the outer layer 60 monotonously decreases from the distal end DE towards the proximal end PE. On the other hand, a ratio of the thickness of the second resin layer 62 relative to the thickness of the outer layer 60 monotonously increases from the distal end DE towards the proximal end PE.
As illustrated in FIG. 1, the first resin layer 61 and the second resin layer 62 have nearly an equal thickness at length-L position away from the distal end DE.
Accordingly, in the outer layer 60, the thickness and the bending rigidity of the first resin layer 61 are dominant over the length-L region continued from the distal end DE. In the region more closer to the proximal end PE, when viewed from the length-L position away from the distal end DE, the thickness and the bending rigidity of the second resin layer 62 are dominant. A portion between the intermediate portion in the axial direction of the outer layer 60 and the proximal end section 17 (see FIG. 4) is configured substantially by the second resin layer 62 only.

The thickness of each resin layer may be calculated, by measuring the thickness of the resin layer at a plurality of points on a cross section of the sheath 16 cut normal to the axial direction thereof (see FIG. 3), and by finding an average value.

While relation between the outer diameter d and length L of the catheter 10 illustrated in FIG. 1 are not specifically limited, length L is preferably 10 times or more and 700 times or less as large as the outer diameter d, and more preferably 50 times or more and 350 times or less. In particular, by adjusting the first resin layer 61 to have a Shore hardness of 20D to 60D, the second resin layer 62 to have a Shore hardness of 65D to 85D, and length L to be 100 times or more and 290 times or less as large as the outer diameter d, the hardness gradation may be well balanced between the kink resistance and pressure response, and thereby bending manipulability and selective insertability into blood vessel of the distal end section 15 may particularly be improved.

On the other hand, in the catheter 10 of the modified example illustrated in FIG. 2, the first resin layer 61 has a thickness larger than those of the second resin layer 62 and the third resin layer 63 in the vicinity of the distal end DE of the sheath 16. The thickness of the third resin layer 63 once gradually increases and then gradually decreases from the distal end side towards the proximal end side of the sheath 16. At the length-L1 position away from the distal end DE, the first resin layer 61 and the third resin layer 63 have the same thickness. In other words, in the outer layer 60, the thickness and the bending rigidity of the first resin layer 61 are dominant, over the range thereof from the distal end DE up to the length-L1 position away therefrom.

At the length-L position away from the distal end DE, the first resin layer 61 and the second resin layer 62 have nearly equal thickness. In this modified example, the third resin layer 63 at the length-L position is formed thicker than the first resin layer 61 and the second resin layer 62. Over the range between the length-L1 position and the length-L position, total of the thickness of the first resin layer 61 and the third resin layer 63, and total of the bending rigidity are dominant.
At the length-L2 position which is located more closer to the distal end than the length-L position, the second resin layer 62 and the third resin layer 63 have nearly equal thickness. In this modified example, relation among the lengths L, L1 and L2 is given as L1<L<L2.
In the outer layer 60, the total thickness and the total bending rigidity of the third resin layer 63 and the second resin layer 62 are dominant, over the range from the length-L position up to the length-L2 position.

On the proximal end side of the length-L2 position away from the distal end DE, the second resin layer 62 which configures the outermost layer is thickest. Length L2 is larger than length L1 . In the outer layer 60, the thickness and the bending rigidity of the second resin layer 62 are dominant, on the base side of the length-L2 position.

Each of the first to third resin layers 61 to 63 has the thickness continuously varied from the distal end side towards the proximal end side of the sheath 16, and the bending rigidity of the outer layer 60 is continuous in the longitudinal direction. Accordingly, the amount of flexion of the distal end section 15 may continuously increase relative to the length of pulling of the operating wire 40, and thereby the distal end section 15 may readily move by a desired amount of flexion.

While relation between the outer diameter d of the catheter 10, and the length lengths L , L1 and L2 is not specifically limited, the length L is preferably 10 times or more and 700 times or less as large as the outer diameter d, and more preferably 50 times or more and 350 times or less.

On the other hand, length L1 is larger than length L, and preferably 5 times or more and 300 times or less as large as the outer diameter d, and is more preferably 20 times or more and 200 time or less.
Length L2 is larger than length L, and preferably 25 times or more and 1400 times or less as large as the outer diameter d, and more preferably 100 times or more and 360 times or less.

Also for the case of the catheter 10 containing the third resin layer 63 according to this modified example (see FIG. 2), it is preferable to configure the first resin layer 61 to have a Shore hardness of 20D to 60D, the second resin layer 62 to have a Shore hardness of 65D to 85D, and to adjust length L to 100 times or more and 290 times or less as large as the outer diameter d. By the adjustment, the hardness gradation may be well balanced between the kink resistance and pressure response, and thereby bending manipulability and selective insertability into blood vessel of the distal end section 15 may particularly be improved.

The catheter 10 of this embodiment additionally has the tubular inner layer 21 having the main lumen 20 formed therein, and a braid layer 50 configured by wires 52 woven around the inner layer 21. The first resin layer 61 is provided in close contact with the braid layer 50. More specifically, in the sheath 16 of this embodiment, the first resin layer 61 configures the innermost layer of the outer layer 60, with a thickness enough to cover the wires 52 of the braid layer 50.

Fluorine-containing thermoplastic polymer material, for example, may be adoptable to the inner layer 21. More specifically, polytetrafluoroethylene (PTFE), polyvinylidene fluoride (PVDF), perfluoroalkoxy (PFA) and so forth may be adoptable. Adoption of the fluorine-containing resin to the inner layer 21 improves the delivery performance of the catheter 10, in the process of delivering a contrast medium, chemical liquid, and so forth through the main lumen 20 to a diseased site.

To the wire 52, metal thin wire composed of stainless steel (SUS) or nickel-titanium alloy, or polymer thin fiber composed of PI, PAI PET, and so forth may be adoptable. Cross-sectional geometry of the wire 52 is not specifically limited, while allowing adoption of round wire and flat wire.

Each sub-lumen 30 having the operating wire 40 laid therethrough is formed so as to extend through the outer layer 60, on the outer circumferential side of the center in the thickness-wise direction. In this embodiment, the sub-lumens 30 are formed through the first resin layer 61 (FIG. 1) or the third resin layer 63 (FIG. 2) so as to extend therethrough, over a predetermined longitudinal range in the vicinity of the distal end DE. On the proximal end side ahead of the middle point of the distal end section 15, the sub-lumens 30 are formed so as to extend through the second resin layer 62. In other words, in the catheter 10 of this embodiment, each sub-lumen 30 having each operating wire 40 laid therethrough is formed in the outer layer 60, and on the outer circumferential side of the braid layer 50.

Each sub-lumen 30 is provided in the longitudinal direction of the catheter 10 (left/right direction in FIGs. 1, 2), and at least the proximal end section 17 (see FIG. 4) of the catheter 10 opens therein.
The sub-lumens 30 of this embodiment have a continuous round tubular geometry, and are formed at a predetermined radial position away from the center of the main lumen 20. As a result of the gradual increase in the thickness of the second resin layer 62 from the distal end side towards the proximal end side, the resin material around the sub-lumen 30 of this embodiment illustrated in FIG. 1 phases from the first resin layer 61 (first resin material 121) into the second resin layer 62 (second resin material 122). On the other hand, in the catheter 10 of the modified example illustrated in FIG. 2, the resin material around the sub-lumen 30 phases from the first resin layer 61 (first resin material 121), through the third resin layer 63 (third resin material 123), into the second resin layer 62 (second resin material 122), when viewed from the distal end side towards the proximal end side.
The sub-lumens 30 of this embodiment are formed in the outer layer 60, so as to give a straight tube form in the axial direction of the sheath 16. The sub-lumens 30 in the present invention may, however, have a geometry other than the straight tube, such as a bent tube spirally wound around the main lumen 20.

Each sub-lumen 30 is a through-hole formed so as to extend through the outer layer 60. The inner wall surface of each sub-lumen 30 of this embodiment is configured by the first to third resin layers 61 to 63. The present invention is, however, not limited thereto, instead allowing embedding of a hollow tube for forming the sub-lumen 30 in the outer layer 60.

The end (distal end) of the operating wire 40 is fixed to the distal end section 15 of the catheter 10. Mode of fixation of the end of the operating wire 40 to the distal end section 15 is not specifically limited. For example, the end of the operating wire 40 may be tied to the marker 66, or may be fused with the distal end section 15 of the sheath 16, or may be adhered to the marker 66 or to the distal end section 15 of the sheath 16 using an adhesive.

The operating wire 40 is composed of a very thin wire material, pulling of the proximal end 41 (see FIG. 4) of which causes bending of the distal end section 15 of the sheath 16. Bending of the sheath 16 in this embodiment includes L-shape folding and bow-like curving.

Materials adoptable to the operating wire 40 used for the present method include polymer fibers composed of polyether ether ketone (PEEK), polyphenylene sulfide (PPS), polybutylene terephthalate (PBT), PI, PTFE and so forth; and flexible metal wires such as SUS, corrosion resistance coated steel wire, titanium, titanium alloy and so forth.
Diameter of the operating wire 40 is preferably 30 to 60 µm. By virtue of such thinness, the operating wire 40 may readily buckle, when it is pushed into the sheath 16. Accordingly, the catheter 10 of this embodiment is substantially exempt from being applied with pressing force in the distal end section 15 thereof, and will not protrude out from the distal end DE even if the operating wire 40 should detach from the distal end section 15.

Representative dimensions of the catheter 10 of this embodiment are typically given below.
Radius of the main lumen 20 is approximately 200 to 300 µm, thickness of the inner layer 21 is approximately 10 to 30 µm, thickness of the outer layer 60 is approximately 100 to 150 µm, and thickness of the braid layer 50 is approximately 20 to 30 µm.
Radial distance between the axial center of the catheter 10 and the center of each sub-lumen 30 is approximately 300 to 350 µm, and inner dimension (diameter) of the sub-lumen 30 is 40 to 100 µm.
Outermost dimension (radius) of the catheter 10 is approximately 350 to 450 µm, and the outer diameter d (see FIG. 2) is smaller than 1 mm. Accordingly, the catheter 10 of this embodiment may be insertable in blood vessels such as celiac artery.

FIG. 4 is a side elevation for explaining operations of the catheter 10 of this embodiment. FIG. 4(a) is a schematic vertical sectional view illustrating the catheter 10 in the natural state, or non-pulled state of the operating wire 40. FIG. 4(b) is a schematic vertical sectional view illustrating the catheter 10 with the operating wire 40 thereof pulled. Note that the individual drawings of FIG. 4 illustrate only one of three operating wires 40 in the catheter 10 of this embodiment.

The proximal end 41 of the operating wire 40 protrudes out from the sub-lumen 30 towards the proximal end side. On the proximal end side of the operating wire 40, there is provided an operating unit 70 for pulling the operating wire 40 so as to bend the distal end section 15 of the catheter 10. Detailed illustration and explanation of a configuration of the operating unit 70 will not be given. The operating unit 70 pulls a single operating wire 40, or a plurality of the operating wires 40 at a time.

In the catheter 10 of this embodiment, tensile force will be applied to the distal end section 15 of the catheter 10, when the proximal end 41 of the operating wire 40 is pulled towards the base side (right in the drawing). If the tensile force is as large as a predetermined level or above, the distal end section 15 will bend from the axial center of the catheter 10 towards the sub-lumen 30 (upward in the drawing) having the pulled operating wire 40 laid therethrough.
By independently controlling the length of pulling of each of three operating wires 40, the distal end section 15 of the catheter 10 may be bent in any directions with a 360 degree circle,. In this way, the direction of advancement of the catheter 10 may freely be controlled only by pulling of the operating wires 40 by the operating unit 70, rather than by torque operation which causes axial rotation of the entire body of the catheter 10. Accordingly, the catheter 10 of this embodiment may be advanced in a desired direction, typically in body cavity such as branched blood vessel.

Now, in the catheter 10 of this embodiment, the thickness of each of the first resin layer 61 and the second resin layer 62, which composes the outer layer 60, continuously varies in the longitudinal direction. The flexibility of the catheter 10 continuously increases from the proximal end PE towards the distal end DE, and conversely the bending rigidity of the catheter 10 increases from the distal end DE towards the proximal end PE. Since the proximal end PE where moment ascribable to the self weight may most heavily be loaded has a large rigidity as described in the above, so that the catheter 10 of this embodiment is now excellent in the geometrical stability, and has a good resilience. On the other hand, since a large flexibility may be obtained in the vicinity of the distal end DE of the catheter 10, the distal end section 15 may be bent by a subtle manipulation through the operating wire 40.
In the catheter 10 of this embodiment, the rigidity of the sheath 16 is continuously varied, by modulating the thickness of the resin layers respectively having different levels of hardness. Accordingly, the sheath 16 may be molded using only a few species of materials.

In the catheter 10 of the modified example, the third resin layer 63, having an intermediate hardness between those of the first resin layer 61 and the second resin layer 62, is formed in between. Accordingly, the first resin layer 61 and the second resin layer 62 may be prevented from separating in the process of molding of the outer layer 60 or in the process of bending manipulation of the catheter 10, even if the first resin layer 61 and the second resin layer 62 were adjusted to have largely different levels of hardness.

In the catheter 10 of this embodiment, the second resin layer 62 having a large hardness is provided on the outer circumferential side of the first resin layer 61. Accordingly, the sheath 16 may be given a good toughness, and thereby the catheter 10 may be improved in the durability.
Since the second resin layer 62 composing the outermost layer of the outer layer 60 has a tackiness smaller than those of the first resin layer 61 and the third resin layer 63, so that the catheter 10 may be given a good manipulability when it is inserted through an endoscope.

In the catheter 10 of this embodiment, the wires 52 composing the braid layer 50 are in close contact with the first resin layer 61. The first resin layer 61 is softer than the second resin layer 62 and the third resin layer 63, and is therefore more adherent to the wires 52. Accordingly, the outer layer 60 and the braid layer 50 may be suppressed from separating from each other.

In the catheter 10 of this embodiment, each sub-lumen 30 having each operating wire 40 laid therethrough is formed on the outer circumferential side of the first resin layer 61. This means that the inner wall surface of each sub-lumen 30, which allows each operating wire 40 to slide therethrough, is configured by the second resin layer 62 or the third resin layer 63 having hardness higher than that of the first resin layer 61, enough to ensure a good slidability of the operating wire 40.

### <Method of Manufacturing Catheter>

A method of manufacturing a catheter of this embodiment (referred to as "the method") will be detailed below.
The method relates to a method of manufacturing the catheter 10 (see FIG. 1) having the sheath 16 which has a main lumen 20, and sub-lumens 30 having a diameter smaller than that of the main lumen 20, both of which being formed in the longitudinal direction so as to extend therethrough; and operating wires 40 laid through the sub-lumens 30 in a slidable manner.
The method includes a body forming step for forming the sheath 16, by co-extruding a molten first resin material (first resin material 121), together with a molten second resin material (second resin material 122) having a hardness higher than that of the first resin material 121 at normal temperature, and having the operating wires 40 introduced therein, from a die 83, while increasing the amount of extrusion D2 of the second resin material 122 relative to the amount of extrusion D1 of the first resin material 121.

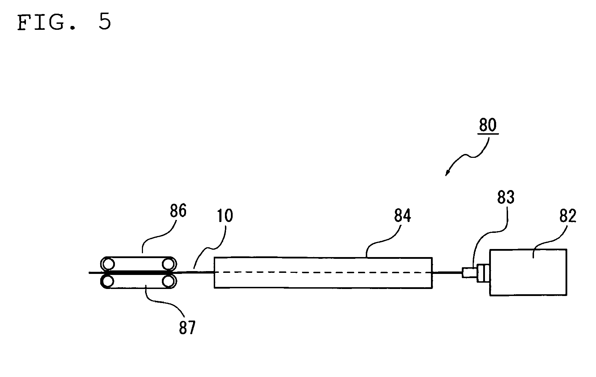

The individual processes of the method will further be detailed below, referring to the attached drawings. FIG. 5 is a drawing illustrating an overall configuration of a manufacturing apparatus 80 for manufacturing the sheath 16 used for the method. Both of the catheter 10 of this embodiment (see FIG. 1) and the modified example (see FIG. 2) may be manufactured using the manufacturing apparatus 80.

The manufacturing apparatus 80 is configured by an extruder 82, a sizer 84, and a take-up device 86 arranged in series.
The extruder 82 is an apparatus configured to accept loading of the first and second resin materials 121, 122, and to extrude the outer layer 60 of the sheath 16 in the form of thin tube, out from the die 83.
The sizer 84 is an apparatus configured to cool the sheath 16 extruded from the extruder 82 so as to give a predetermined diameter, to which a water tank may be adoptable for example.
The take-up device 86 is composed of rollers driven in an opposed manner, and is configured to take up the end of the sheath 16, extruded from the extruder 82 and then cooled by the sizer 84, at a predetermined take-up speed.
In the manufacturing apparatus 80, the sheath 16 to be molded is adjusted to have a predetermined diameter, by adjusting parameters such as rate of taking-up by the take-up device 86, rate of cooling by the sizer 84, distance between the die 83 and the sizer 84, and so forth.

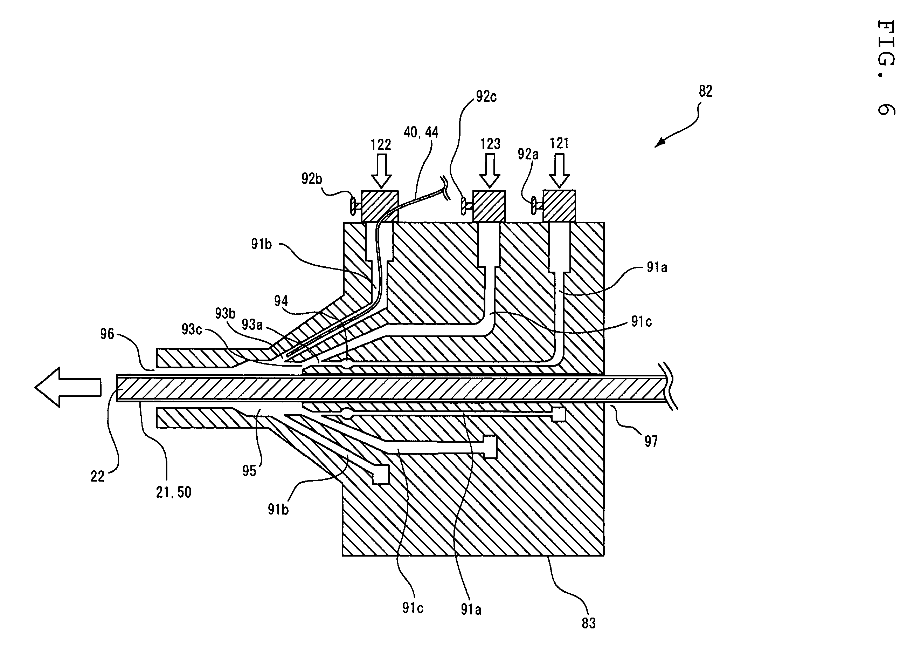

FIG. 6 is a schematic vertical sectional view illustrating the extruder 82. A method of manufacturing the catheter 10, illustrated in FIGs. 2 and 3, using the extruder 82 will be explained below.
In the body forming step of the present method, the first to third resin materials 121 to 123, which serve as major constituents of the sheath 16, are extruded around the core wire 22 to form the sheath 16.
The core wire 22 is a mandrel formed into a cylindrical geometry, and is a component used for forming the main lumen 20, while being drawn out from the sheath 16.

The core wire 22 optionally has a mold releasing treatment on the circumferential surface thereof. The mold releasing treatment may be given by coating a mold releasing agent such as fluorine-containing one or silicone-based one, or by optical or chemical surface treatment.
While materials for composing the core wire 22 are not specifically limited, metal materials may preferably be used from the viewpoint of large tensile strength and corrosion resistance. More specifically, copper or copper alloy; alloy steels such as carbon steel, stainless steel (SUS) and so forth; and nickel or nickel alloy may be exemplified.

Prior to the body forming step, the circumferential surface of the core wire 22 is preliminarily given the inner layer 21 and the braid layer 50 formed therearound.
In the body forming step, the first to third resin materials 121 to 123 are extruded onto the surface of the braid layer 50, and thereby the outer layer 60 is formed. As a consequence, the braid layer 50 is contained in the outer layer 60 as illustrated in FIG. 2.

The extruder 82 has the die 83, and flow control valves 92a to 92c.
The die 83 has three lines of flow paths 91a to 91c through which the first to third resin materials 121 to 123 in molten states are respectively extruded.
The flow control valves 92a to 92c may increase or decrease the flow rates of the first to third resin materials 121 to 123 at desirable levels.
The die 83 has a through-hole 97, through which the core wire 22 is inserted, formed at the axial center thereof so as to extend therethrough. The core wire 22 is fed into the through-hole 97 at a predetermined speed. Direction of feeding of the core wire 22 is leftward in the drawing, as indicated by an arrow.

The flow path 91a has an expanding diameter portion 94 formed on the upstream side of an output port 93a in the vicinity thereof. By virtue of provision of the expanding diameter portion 94 in the flow path 91a, the amount of extrusion D1 of the first resin material 121 may be stabilized. Accordingly, the braid layer 50 provided on the surface of the core wire 22 may stably be covered with the first resin material 121.
While FIG. 6 exemplifies the die 83 having the expanding diameter portion 94 formed only in the flow path 91a, a cavity may be formed also in the flow paths 91b and 91c, similarly on the upstream side of the output ports 93b and 93c in the vicinity thereof.

The output port 93a of the flow path 91a is provided on the inner circumferential side closest to the surface of the core wire 22. On the outer circumferential side of the output port 93a, the output port 93c of the flow path 91c, and the output port 93b of the flow path 91b are provided in this order.
Accordingly, the first resin material 121 discharged through the output port 93a is extruded onto the outer circumferential surface of the core wire 22. The third resin material 123 is discharged through the output port 93c onto the outer circumferential surface of the first resin material 121, and the second resin material 122 is discharged further thereon through the output port 93b.

The first to third resin materials 121 to 123 discharged through the flow paths 91a to 91c form a three-layered stack in a cavity 95, then shaped by a nozzle 96 at the end of the die 83 so as to give a predetermined thickness, and coated on the surface of the core wire 22. The first to third resin materials 121 to 123 extruded out from the nozzle 96 are cooled and cured, and respectively form the first to third resin layers 61 to 63. In short, by extruding the first to third resin materials 121 to 123 in a three-layered manner out through the nozzle 96, the outer layer 60 is formed over the surface of the core wire 22.

Out from the die 83, the operating wire 40 is extruded together with the resin materials. More specifically, as illustrated in FIG. 6, out from the die 83 of this embodiment, the operating wire 40 is extruded together with the second resin material 122 through the flow path 91b.
In this way, the operating wires 40 laid in the longitudinal direction of the sheath 16 are embedded in the outer layer 60.

### (Body Forming Step)

In the body forming step of the present method, while increasing the amount of extrusion D2 of the second resin material 122 relative to the amount of extrusion D1 of the first resin material 121, the first to third resin materials 121 to 123 are extruded so as to mold the sheath 16. The amount of extrusion of the resin material herein means a volumetric flow rate of the resin material extruded out from the nozzle 96 of the die 83 per unit time.

In the body forming step, the outer layer 60 may be molded from the distal end side towards the proximal end side, or from the proximal end side towards the distal end side. In the present method, an exemplary case of extrusion from the distal end side towards the proximal end side will be explained.
As illustrated in FIG. 2, in the outer layer 60 of the catheter 10 of this embodiment, when viewed from the distal end DE (left in the drawing) towards the proximal end PE (right in the drawing), the first resin layer 61 gradually decreases, whereas the second resin layer 62 gradually increases. In a similar view, the third resin layer 63 gradually increases and then decreases. Assuming a position distant by a predetermined length away from the distal end DE as a boundary, each of the first to third resin layers 61 to 63 has an approximately uniform thickness on the proximal end PE side of the boundary position.

In the body forming step, the amounts of extrusion D1 to D3 of the first to third resin materials 121 to 123 are adjusted typically as described below, by open/close operation of the flow control valves 92a to 92c of the die 83.
(1) While setting an initial condition to D2=D3≈0, the first resin material 121 only is extruded from the nozzle 96 so as to mold the distal end DE (initial extrusion process).
(2) Following the initial extrusion process, the first to third resin materials 121 to 123 are extruded out through the nozzle 96 over a predetermined extrusion length L1 (see FIG. 2), while gradually decreasing D1, and gradually increasing D2 and D3 (preliminary extrusion step). In the final stage of the preliminary extrusion step, a relation of D1≈D3>D2 holds.
(3) Following the preliminary extrusion step, the first to third resin materials 121 to 123 are extruded out through the nozzle 96, while further gradually decreasing D1, gradually increasing D2, and turning D3 from gradual increase into gradual decrease. At a point of extrusion length L (see FIG. 2), a relation D3>D1≈D2 holds. Furthermore, the first to third resin materials 121 to 123 are extruded out through the nozzle 96 up to the extrusion length L2 (see FIG. 2), while gradually decreasing D1 and D3, and gradually increasing D2, so as to mold the distal end section 15 (first extrusion process). In the final stage of the first extrusion process, a relation of D2≈D3>D1 holds.
(4) Following the first extrusion process, D1 and D3 are further gradually decreased, and D2 is further gradually increased. While setting the condition to D1=D3≈0, the second resin material 122 only is then extruded out through the nozzle 96, so as to mold the outer layer 60 up to the proximal end PE (second extrusion process).

The amounts of extrusion D1 to D3 may be adjustable by speed of drawing of the core wire 22 by the take-up device 86 (see FIG. 5), aperture of the flow control valves 92a to 92c of the die 83, or feeding pressure of the first to third resin materials 121 to 123 to the flow paths 91a to 91c.

By the extrusion process described in the above, the first, third, second resin layers 61, 63, 62 are stacked in this order from the bottom to the top over the circumferential surface of the core wire 22, and thereby the outer layer 60 is molded.

In short, according to the present method, the first extrusion process takes place, after the preliminary extrusion step in which the molten third resin material 123, having the operating wire 40 introduced therein, is extruded together with the molten second resin material 122 from the die 83.
In the first extrusion process, the first to third resin materials 121 to 123 are extruded together from the die 83 so as to mold the sheath 16, while increasing the amount of extrusion D2 of the second resin material 122, relative to the amounts of extrusion D1, D3 of the first and third resin materials 121, 123.

Although the second resin layer 62, in the preliminary extrusion step of the present method, is formed to give a thin layer in the vicinity of the distal end DE of the sheath 16, while adjusting the amount of extrusion D2 of the second resin material 122 to not-zero, the present invention is not limited thereto. Alternatively, in the preliminary extrusion step, only the first resin material 121 and the third resin material 123 may be extruded.

For the case where the catheter 10 (see FIG. 1) having the outer layer 60 composed of two layers, which are the first and second resin layers 61, 62, is manufactured, open and close operations of the flow control valves 92a, 92b of the die 83 may be as the following.
In the initial (preliminary) extrusion process, only the first resin material 121 is extruded over a predetermined length.
Thereafter, in the first extrusion process, the distal end section 15 is extrusion-molded while gradually decreasing the amount of extrusion D1 of the first resin material 121, and gradually increasing the amount of extrusion D2 of the second resin material 122. At the time correspondent to extrusion length L, a relation D1≈D2 holds.
Following the first extrusion process, only the second resin material 122 is extruded out through the nozzle 96 while setting D1≈0, so as to mold, by extrusion, the outer layer 60 up to the proximal end PE (second extrusion process).

The operating wire 40 is embedded in the surficial portion of the outer layer 60, while being laid through the flow path 91b, and guided by the second resin material 122.
In the preliminary molding process, the operating wire 40, introduced through the flow path 91b and having the end thereof reached the cavity 95, is extruded out through the nozzle 96, while being covered therearound with the third resin material 123. Then in the first extrusion process and the second extrusion process, the operating wire 40 is consequently extruded out through the nozzle 96, while being covered therearound with the second resin material 122.
Accordingly, as illustrated in FIG. 2, the operating wire 40 is embedded in the third resin layer 63 in the vicinity of the distal end DE of the outer layer 60 of this embodiment, whereas in the second resin layer 62 in the proximal end PE side.

Now in the body forming step of the present method, close adherence between the operating wire 40 and the outer layer 60 may be prevented, by providing a clearance between the operating wire 40, and the second or third resin material 122, 123 in the process of extrusion molding of the outer layer 60. Accordingly, in the catheter 10 of this embodiment, slidability between the operating wires 40 and the sub-lumens 30 may be ensured.

Method of providing the clearance between the operating wire 40, and the second or third resin material 122, 123 is not specifically limited. In an exemplary method adoptable herein, the operating wire 40 may be inserted through the flow path 91b, while being covered with a filler material 44 provided so as to surround it in the radial direction, and then extruded together with the third resin material 123 or the second resin material 122. Liquid or soluble solid may be adoptable to the filler material 44. Solid filler material 44 may be exemplified by water-soluble or solvent-soluble solid materials, and more specifically by polyvinyl alcohol (PVA), partially saponified product of polyvinyl acetate, polyacrylic acid, polyacrylamide, polyethylene oxide, and biodecomposable resin such as starch.
Then in the body forming step, by removing the filler material 44 from the surface of the operating wire 40 embedded in the outer layer 60, the sub-lumen 30 may be formed in the outer layer 60 so as to extend therethrough, and thereby the sub-lumen 30 and the operating wire 40 are made slidable relative to each other.

Another possible method of providing the clearance between the operating wire 40, and the second or third resin material 122, 123, may be such as extruding the second resin material 122 while blowing a highly pressurized air around the radial circumference of the operating wire 40.

### (Other Processes)

From the sheath 16 having the operating wires 40 laid through the outer layer 60 in a slidable manner, the core wire 22 is drawn out so as to form the main lumen 20. The core wire 22 having a mold releasing treatment shows an interfacial strength effected on the inner layer 21, which is smaller than separation strength of the outer layer 60 effected on the braid layer 50 or on the inner layer 21. For this reason, by drawing the core wire 22 out from the sheath 16, the inner layer 21 and the braid layer 50, having been formed on the circumferential surface of the core wire 22, are left inside the sheath 16.

Next, the marker 66 is press-fastened on the outer circumference of the distal end section 15 of the sheath 16, and the end of the operating wire 40 is fixed to the marker 66. Then a hydrophilic coated layer 64 is formed on the outer circumference of the outer layer 60, over a partial length on the distal end side thereof.
The catheter 10 of this embodiment may be obtained in this way.

Note that the present invention is not limited to the above-described embodiment, instead allowing various modifications and improvement, so long as the object of the present invention may be accomplished.

### <Modified Example>

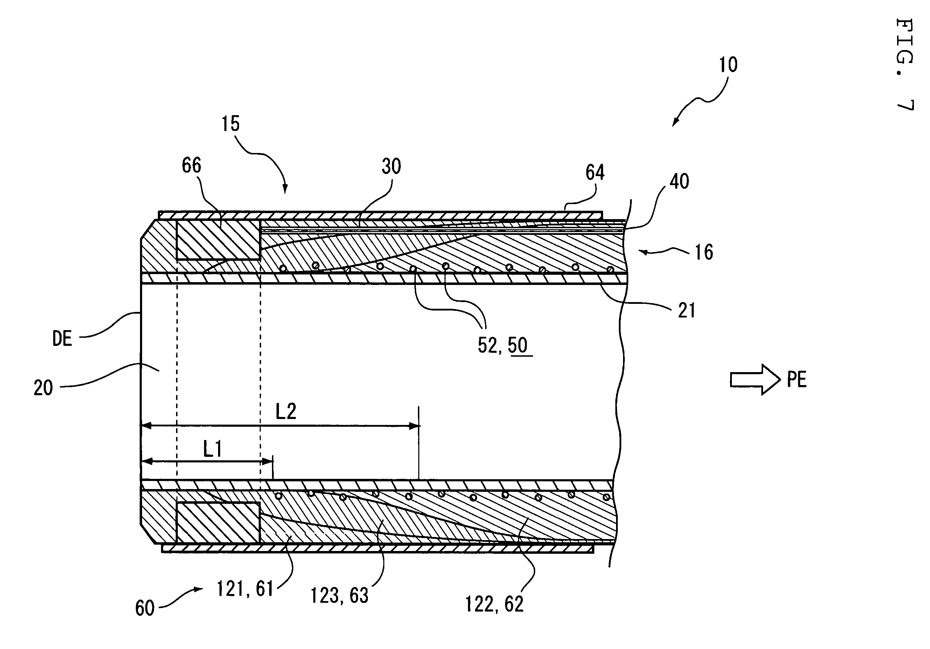

FIG. 7 is a partial schematic vertical sectional view illustrating the catheter 10 of a modified example. The drawing illustrates a cross section of the end portion of the catheter 10 cut in the longitudinal direction.
In this modified example, the first resin layer 61 is provided on the outer circumferential side of the second resin layer 62, over a partial length of the sheath 16 on the distal end DE side thereof.

In the catheter 10 of this modified example, the second resin layer 62 is formed on the inner side of the first resin layer 61. More specifically, the first resin layer 61, the third resin layer 63 and the second resin layer 62 are stacked in this order when viewed from the outer circumferential side, so as to form the outer layer 60.

The second resin layer 62 has a uniform thickness in the vicinity of the distal end DE of the sheath 16, and gradually increases beyond a middle point of the length of the distal end section 15.
The thickness of the third resin layer 63 once gradually increases and then gradually decreases from the distal end side towards the proximal end side of the sheath 16.
The thickness of the first resin layer 61 decreases from the distal end side towards the proximal end side of the sheath 16.

The first resin layer 61 is thickest over the range from the distal end DE towards the length-L1 position, so that the thickness and bending rigidity of the first resin layer 61 are dominant in the outer layer 60.
On the other hand, the second resin layer 62 and the third resin layer 63 have nearly same thickness at the position distant by length L2 (>L1) from the distal end DE.
The second resin layer 62 is thickest over the range from the position distant by length L2 from the distal end DE towards the proximal end PE, so that the thickness and bending rigidity of the second resin layer 62 are dominant in the outer layer 60.

In short, the outer layer 60 of this modified example has the same profile of thickness of the first to third resin layers 61 to 63 in the above-described embodiment.

The first and third resin layers 61, 63 are stacked over a partial length in the vicinity of the distal end DE, and the outer circumferential surface thereof is covered with the hydrophilic coated layer 64.
Accordingly, to the outer surface of the sheath 16 which exposes out from the coated layer 64 on the proximal end side, only the second resin layer 62 exposes. In this way, the first and third resin layers 61, 63, having smaller hardness as compared with the second resin layer 62, are protected by the coated layer 64.

In this modified example, the end of an operating wire 40 is surrounded by the first resin material 121. The resin material around the operating wire 40 continuously phases from the first resin material 121, the third resin material 123, and to the second resin material 122, when viewed from the end to the base.

In this modified example, the first resin material 121, which is a resin material having the smallest hardness, is stacked in the outermost circumferential portion of the outer layer 60. In other words, in this modified example, the soft first resin material 121 is stacked on the outer circumference farthest from the center of rigidity, or the axial center of the main lumen 20, in the vicinity of the distal end DE. Therefore, bendability of the sheath 16 when the operating wire 40 is pulled may be improved as compared with the above-described embodiment.

While the above-descried embodiment exemplified a mode having the sub-lumen 30 formed outside the braid layer 50, the present invention is not limited thereto. More specifically, the sub-lumens 30 may be formed inside the inner layer 21, and the braid layer 50 may be provided therearound.
In addition, the inner layer 21 may have a dominant thickness in the sheath 16, and the inner layer 21 may be configured by a stacked structure of the first to third resin layers 61 to 63.
By virtue of protection of the outer circumference of the sub-lumens 30 with the braid layer 50 as described in the above, the operating wire 40 will not break through the catheter 10 to be exposed externally, even if an excessive tensile force should be applied to the operating wire 40 during manipulation.

On the other hand, in the present method, by adjusting the take-up speed by the take-up device 86 (see FIG. 5) or extrusion speed of the first to third resin materials 121 to 123, the total thickness of the sheath 16 may be increased from the distal end DE side towards the proximal end PE side.
In this way, a sufficient level of bending strength of the proximal end section 17 of the catheter 10, where the largest bending moment is loaded, may be obtained while ensuring a sufficient level of bendability in the distal end section 15 of the catheter 10.

While the catheter 10 of this embodiment have three operating wires 40 laid through the sub-lumens 30 one-by-one in a slidable manner, the present invention is not limited thereto. The number of operating wires 40 may be one, two, or four or more. The number of sub-lumens 30 may be same with, or different from, the number of operating wires 40. More specifically, a single sub-lumen 30 may have a plurality of operating wire 40 laid therethrough, or a sub-lumen 30 having no operating wire 40 laid therethrough may be provided to the sheath 16.

This application claims priority right based on Japanese Patent Application No. 2009-054806 filed on March 9, 2009, the entire content of which is incorporated hereinto by reference.

## Claims

1. A catheter comprising:
a tubular body comprising a main lumen, and a sub-lumen having a diameter smaller than that of the main lumen, both of which being formed in a longitudinal direction so as to extend therethrough; and
an operating wire laid through the sub-lumen in a slidable manner, with an end thereof fixed to a distal end section of the tubular body,
the tubular body being configured by a first resin layer and a second resin layer having a hardness higher than that of the first resin layer, each of which being composed of a resin material, stacked in a thickness-wise direction, with a thickness of the second resin layer increased from a distal end side towards a proximal end side.

2. The catheter according to Claim 1,
wherein the thickness of the first resin layer monotonously decreases from the distal end side towards the proximal end side of the tubular body.

3. The catheter according to Claim 1 or 2,
further comprising a third resin layer, having an intermediate hardness between those of the first resin layer and the second resin layer, stacked between the first resin layer and the second resin layer.

4. The catheter according to any one of Claims 1 to 3,
wherein the second resin layer is provided in the outer circumferential side of the first resin layer.

5. The catheter according to Claim 4,
wherein the resin material composing the second resin layer has a tackiness smaller than that of the resin material composing the first resin layer.

6. The catheter according to any one of Claims 1 to 5,
further comprising a tubular inner layer having the main lumen formed therein, and a braid layer configured by wires woven around the inner layer,
wherein the first resin layer is formed in close contact with the braid layer.

7. The catheter according to any one of Claims 1 to 6,
wherein the sub-lumen having the operating wire laid therethrough is formed so as to extend on the outer circumferential side of the first resin layer.

8. The catheter according to any one of Claims 1 to 3,
wherein the first resin layer is provided on the outer circumferential side of the second resin layer, over a partial length of the distal end side of the tubular body.

9. A method of manufacturing a catheter which has a tubular body comprising a main lumen, and a sub-lumen having a diameter smaller than that of the main lumen, both of which being formed in a longitudinal direction so as to extend therethrough; and an operating wire laid through the sub-lumen in a slidable manner, the method comprising:
a body forming step for forming the tubular body, by co-extruding a molten first resin material, together with a molten second resin material having a hardness higher than that of the first resin material at normal temperature, and having the operating wire introduced therein, from a die, while increasing the amount of extrusion of the second resin material relative to the amount of extrusion of the first resin material.

10. The method of manufacturing a catheter according to Claim 9, configured to execute the body forming step, after a preliminary extrusion step in which a molten third resin material having the operating wire introduced therein was co-extruded from the die together with the molten second resin material,
wherein, in the body forming step, the tubular body is formed by co-extruding the first, second and third resin materials from the die, while increasing the amount of extrusion of the second resin materials relative to the amount of extrusion of the first and third resin material.
